# EUROPEAN PATENT APPLICATION

(11) **EP 0 930 019 A2**
(43) Date of publication of application: **21.07.1999**
(21) Application number: 98124707.5
(22) Date of filing: 24.12.1998
(51) Int. Cl.: A23L 1/30, A61K 35/78

(54) **Composition for treating obesity and foods and drinks containing the same**

(30) Priority: 25.12.1997 JP 35787397
(71) Applicant: LOTTE CO., LTD, Shinjuku-ku Tokyo (JP)
(72) Inventor: Inoue, Shuji, Kanazawa-ku, Yokohama-shi, Kanagawa (JP); Yamamoto, Manabu, Urawa-shi, Saitama (JP); Shimura, Susumu, Hachioji-shi, Tokyo (JP)
(74) Representative: Reitzner, Bruno, Dr.

(57) **Abstract**

An extract from *Cassia mimosoides* L. var. *nomame* Makino classified into Leguminosac is admixed with an extract from mulberry classified into Moraceae, wherein the extract from *Cassia mimosoides* L. var. *nomame* Makino is effective to inhibit an absorption of fats whilst the other extract from mulberry is effective to inhibit an absorption of sugars, thereby providing enhancement effects of both reducing a weight of human body and suppressing an increase in weight of human body in addition of a remarkable reduction of fats accumulated around viscera.

## Description

The present invention relates to a composition effective for improvement and treatment of obesity and fat cells, and foods and drains containing the same composition.

In recent years, diets have been changed into fatty meals and thus the number of obese persons has greatly been increased thereby to raise an object of the public concern.

An excess intake energy over the consumption energy may be accumulated in human body as fats. In order to avoid the accumulation of the fats in human body, it is effective to go on a Rapid reducing and extremely lower calorific diet. Since the diet may apply an extensive stress to the human body so that the health breaks down.

It has been well-known that the obesity may cause various diseases of adult peoples such as diabetes, arteriosclerosis or hypertension. The fats accumulated in the body may be classified into two types, one is the fat accumulated under a skin and other is the fat accumulated around the viscera. It has been recognized that the fat accumulated around the viscera is closely related to the obesity-complication, which has been reported by a literature entitled "obesity : diagnosis / treatment / guidance" published in 1993.

In the compositions of the foods, triglyceride is the highest calorie fat. An excess intake of such fats causes the obesity. The fats may chemically be decomposed by pancreatic lipase to be absorbed into the small intestine. In order to prevent the absorption of the fats into the small intestinc, it is effective to inhibit the activity of pancreatic lipase, for which reason some medicines as the pancreatic lipase inhibitor, for example, tetrahydroribstatine had been developed. Such medicines are used for medical treatment to the obesity even adverse effects may appear. Normal peoples should never usually and daily intake those medicines to prevent the obesity. Natural composition or substance may be used in daily file in view of the safety.

In Agric Biol. Chem., 37, 1225 (1973), it is disclosed that soybean protein may be used as a natural lipase inhibitor. In nutrition Reports International, 32, 1107 (1985), it is disclosed that grain protein may also be used as other natural lipase inhibitor. In J. Food Sci., 49, 956 (1984), it is disclosed that hemi-cellulose may also be used as other natural lipase inhibitor. In Am. J. Clin. Nutr., 42, 629 (1985), it is disclosed that wheat bran may also be used as other natural lipase inhibitor. In Report, Department of Agriculture of Meiji University 73, 9 (1986), it is disclosed that phosphortizircholine of radish seeds. In J. Food Sci., 53, 250 (1988), it is disclosed that myoinositol may also be used as other natural lipase inhibitor. In Japanese laid-open patent publication No. 1-102022, it is disclosed that a composition of Oolong tea may also be uscd as other natural lipase inhibitor. In Japanese laid-open patent publication No. 3-219872, it is disclosed that an extract by water from sweet paper, pumpkin, *Tricholoma conglobatum* Sacc, *Grifola frondosa* Dickson, *Hijikia fusiformis* Harv. Okamura, green teas, red teas, and Oolong teas. Those natural lipase inhibitors have not yet been made practicable.

In Japanese patent No. 2602387, it is disclosed that further lipase inhibitors are extracts from roots of *A. pubesens* Maxim, *A. offoconarum* HANCE, bark of *Myrica rubra* Sieb. et Zucc, *Cassia mimosoides* L. var. *nomame* Makino = *Cassia nomame* Honda and *Cassia* Semen. In Japanese patent No. 2628832, it is disclosed that two novel tannins are contained as the effective components in *Cassia mimosoides L.* var. *nomame* Makino. In Japanese laid-open patent publication No. 8-259557, it is disclosed that flavane oligomers are also effcctive components of *Cassia mimosoides* L. var. *nomame* Makino.

Needless to say, it is effective for prevention and improvement of the obesity to suppress the absorption of triglyceride. Notwithstanding, excess intakes of starch or sugars may also cause the obesity.

Extracts from Gymnema-sylvestris R. Br and Gymnema acids have been known as the natural inhibitor to absorption of the sugars.

In Biochem. Biophys. Acta. 658, 387 (1981), it is disclosed that an extract from wheat flour may be used as a natural inhibitor effective to a sugar-digestive enzyme due to inhibiting human saliva α-amylase. In FEBS Lett,, 309(1) 68 (1992), it is disclosed that an extract from rice bran may be used as other natural inhibitor effective to a sugar-digcstive enzyme due to inhibiting human saliva α-amylase. In Agric. Biol. Chem., 54(8), 1939(1990), it is also disclosed that catechins and theaflavins of tea may be used as other natural inhibitor effective to a sugar-digestive enzyme due to inhibiting human saliva α-amylase. In Japanese laid-open patent publication No. 5-148153, it is disclosed that deoiling and non-deoiling powder of laurel may be used. In Japanese laid-open patent publication No. 4-27839, it is disclosed that an extract from laurel may also be used. In Japanese patent publication No. 60-36746 and in Japanese laid-open patent publication No. 7-59539, it is disclosed that the Guava leaf may be used.

In Japanese laid-open patent publication No. 53-44530 and also Agric. Biol. Chem., 52, 121(1988), it is disclosed that 1-deoxynodirimycin contained in mulberry is capable of inhibiting the activity of α-glucosidase as sugar-digestive enzyme whereby digestion of sugars and these absorption into the intestine are delayed. In Japanese laid-open patent publication No. 9-140351, it is disclosed that an extract from mulberry leaf including 1-deoxynodirimycin and foods and drinks composition as blood sugar improver including flavonoid may be used as the α -glucosidase inhibitors.

In the meantime, in order to improve the obesity and reduce the fats in the human body, it is ideal to suppress absorptions of both the fats and sugars. However, no chemical substance effective to suppress absorptions of both the fats and sugars has been developed yet or found.

In the above circumstances, it had been required to develop a novel composition effective to suppress absorptions of both triglyceride as a fat and sugars for effective improvement in obesity and possible reduction in fats in human body, wherein the composition may be used in foods and drinks for daily dietary life to prevent excess intakes of fats and sugars as well as to develop novel safe foods and drinks containing the composition.

Accordingly, it is an object of the present invention to provide a novel composition free from the above problems.

It is a further object of the present invention to provide a novel composition effective to suppress absorptions of both triglyceride as a fat and sugars for effective improvement in obesity and possible reduction in fats in human body.

It is a still further object of the present invention to provide a novel composition may be used in foods and drinks for daily dietary life to prevent excess intakes of fats and sugars.

It is yet a further object of the present invention to provide novel foods and drinks containing the composition effective to suppress absorptions of both triglyceride as a fat and sugars for effective improvement in obesity and possible reduction in fats in human body.

It is a further more object of the present invention to provide novel safe foods and drinks for daily dietary life to prevent excess intakes of fats and sugars.

The above and other objects, features and advantages of the present invention will be apparent from the following descriptions.

It has been confirmed by the inventors that the use of an extract from *Cassia mimosoides* L. var. *nomame* Makino classified into Leguminosae, which is effective to inhibit an absorption of fats, in combination with an extract from mulberry classified into Moraceae, which is effective to inhibit an absorption of sugars may provide enhancement effects of both reducing a weight of human body and suppressing an incrcase in weight of human body in addition of a remarkable reduction of fats accumulated around viscera.

In accordance with the present invention, an extract extracted with water or organic solvents from *Cassia mimosoides* L. var. *nomame* Makino classified into Leguminosac is used in combination with an extract extracted with water or organic solvents from mulberry classified into Moraceae, wherein the extract from *Cassia mimosoides* L. var. *nomame* Makino is effective to inhibit an absorption of fats whilst the other extract from mulberry is effective to inhibit an absorption of sugars, thereby providing enhancement effects of both reducing a weight of human body and suppressing an increase in weight of human body in addition of a remarkable reduction of fats accumulated around viscera.

Alternatively, a roasted extract with reduced astringency and bitterness, which has been extracted with water or organic solvents from *Cassia mimosoides* L. var. *nomame* Makino classified into Leguminosac is used in combination with an extract extracted with water or organic solvents from mulberry classified into Moraceae, wherein the roasted extract from *Cassia mimosoides* L. var. *nomame* Makino is effective to inhibit an absorption of fats whilst the other extract from mulberry is effective to inhibit an absorption of sugars, thereby providing enhancement effects of both reducing a weight of human body and suppressing an increase in weight of human body in addition of a remarkable reduction of fats accumulated around viscera.

The composition in accordance with the present invention is effective to reduce a weight of human body and suppressing an increase in weight of human body in addition of a remarkable reduction of fats accumulated around viscera. This composition is extracted from natural substances, for which reason the composition may be used in daily file in view of the safety. This composition is further reduced in astringency and bitterness for addition into foods and drinks.

The first present invention provides a composition comprising an admixture of a first extract from *Cassia mimosoides* L. var. *nomame* Makino and a second extract from mulberry.

It is preferable that a ratio of admixing in weight of the first extract and the second extract is in the range of 1 : 0.5 to 1 : 2.5.

It is also preferable that the first extract is roasted before admixed with the second extract.

It is further preferable that the first extract is roasted at 100-130°C for 1-5 hours.

The second present invention provides foods and drinks containing a composition comprising an admixture of a first extract from *Cassia mimosoides* L. var. *nomame* Makino and a second extract from mulberry.

It is preferable that the composition is admixed into a base material for the foods and drinks.

It is also preferable that a ratio of admixing in weight of the first extract and the second extract is in the range of 1 : 0.5 to 1 : 2.5.

It is also preferable that the first extract is roasted before admixed with the second extract.

It is further preferable that the first extract is roasted at 100-130°C for 1-5 hours.

The third present invention provides an obesity-improving agent including a composition comprising an admixture of a first extract from *Cassia mimosoides* L. var. *nomame* Makino and a second extract from mulberry.

It is preferable that a ratio of admixing in weight of the first extract and the second extract is in the range of 1: 0.5 to 1 : 25.

It is also preferable that the first extract is roasted before admixed with the second extract.

It is further preferable that the first extract is roasted at 100-130°C for 1-5 hours.

The fourth present invention provides a method of preparing a composition comprising the steps of : preparing a first extract from *Cassia mimosoides* L. var. *nomame* Makino and a second extract from mulberry ; and admixing the first and second extracts.

It is preferable that the first and the second extracts are admixed at a ratio in weight in the range of 1 : 0.5 to 1 : 2.5.

It is also preferable to further comprise the step of roasting the first extract before admixed with the second extract.

It is further preferable that the first extract is roasted at 100-130°C for 1-5 hours.

The fifth present invention provides a method of preparing foods and drinks comprising the steps of : preparing a first extract from *Cassia mimosoides* L. var. *nomame* Makino and a second extract from mulberry ; admixing the first and second extracts to prepare a composition ; and adding the composition into foods and drinks.

It is preferable that the composition is admixed into a base material for the foods and drinks.

It is also preferable that the first and the second extracts are admixed at a ratio in weight in the range of 1 : 0.5 to 1 : 2.5.

It is also preferable to further comprise the step of roasting the first extract before admixed with the second extract.

It is also preferable that the first extract is roasted at 100-130°C for 1-5 hours.

The sixth present invention provides a method of preparing an obesity-improving agent comprising the steps of : preparing a first extract from *Cassia mimosoides* L. var. *nomame* Makino and a second extract from mulberry ; admixing the first and second extracts ; forming the admixture into an obesity-improving agent.

It is preferable that the first and the sccond extracts are admixed at a ratio in weight in the range of 1 : 0.5 to 1 : 2.5.

It is also preferable to further comprise the step of roasting the first extract before admixed with the second extract.

It is also preferable that the first extract is roasted at 100-130°C for 1-5 hours.

The above extract may be extracted with water or polarized or unpolarized solvent from *Cassia mimosoides* L. var. *nomame* Makino. The method of extraction should not be limited to these methods. A dried above-ground part of *Cassia mimosoides* L. var. *nomame* Makino is cut into fine pieces or crushed with a mill for subsequent extraction with an organic solvent, for example, water, methanol, ethanol, ethyl acetate, acetone, or mixture thereof, so as to obtain an extract in the form of paste or powder of light brown color or green brown color. This extract may be added directly into foods and drinks. Alternatively, this cxtract may also be processed with chromatography for fractionation prior to addition into the foods and drinks. Further, alternatively, the cxtract from *Cassia mimosoides* L. var. *nomame* Makino may be roasted to reduce the astringency and bitterness prior to addition into the foods and drinks. It is preferable to roast the extract or fractionated extract under conditions at a temperature of 100-130°C for 1-5 hours, preferably at a temperature of 110°C for 3 hours, whereby the extract is reduced in astringency and bitterness as well as unpleasant odors without reduction of lipase-inhibition activity.

The other extract from mulberry which includcs 1-deoxynodirimycin as the effective component may be prepared in accordance with the method disclosed in Japanese laid-open patent publication No. 9-140351.

Alternatively, the dried powder of mulberry leaf is subjected to water or an organic solvent or mixture thereof to obtain an extract from the mulberry leaf. The method of extraction should not be limited to these methods. The obtained extract is in the form of paste or powder of light brown color or green brown color. This extract may be added directly into foods and drinks. Alternatively, this extract may also be processed with chromatography for fractionation prior to addition into thc foods and drinks. Further, alternatively, the extract from mulberry may be roasted. A condensed mulberry-extract solution is added with dextrin as excipient at 15-18%, preferably 20% for subsequent spray drying proccss. The addition of excipient into the condensed mulberry-extract solution result in flavor-improved and fine-particle extract which is suitable for addition into the foods and drinks.

The use of the extract from *Cassia mimosoides* L. *var. nomame* Makino in combination with the extract from mulberry is effective to reduce a weight of human body and suppress an increase in weight of human body in addition of a remarkable reduction of fats in the human body particularly accumulated around viscera. A weight ratio in mixing of the extract from *Cassia mimosoides* L. var. *nomame* Makino to the extract from mulberry may be varied, preferably in the range of 1 : 0.5 to 1 : 2.5, wherein an amount or weight of excipient is excluded from the extract from mulberry.

The admixture of the extract from *Cassia mimosoides* L. var. *nomame* Makino with the extract from mulberry may be used as the composition to be added into foods and drinks as the ohesity improver after dilution with a proper solvent or condensation. This composition may be used in the form of paste or powder with suction or frceze-drying operation. The composition may be filled into capsules or soft capsules. Alternatively, the composition may also be formed in tablet type or granular type to be taken as the obesity-preventive agent. Further, alternatively, the composition may also be mixed into materials for all foods and drinks, for example, various confectioneries such as chewing gums, chocolates, candies, tablets, jelly, biscuits, cookies, crackers and snacks, or various iced confectioneries such as ice creams and frozen confectioneries, various drinks such as soft drinks, nutrition drinks, cosmetic drinks and teas, and further breads, hams, soups, spaghettis and frozen foods.

### EXAMPLE 1 :

40 kg of crushed above-ground parts of *Cassia mimosoides* L. var. *nomame* Makino was immersed into 200 liters of 50%-ethanol solution for subsequent stirring operation at 60°C for 2 hours. A first remainder after the first filtration operation was added with 150 liters of 50%-ethanol solution for subsequent further stirring operation at 60°C for 2 hours. A second remainder after the second filtration operation was further added with 150 liters of 50%-ethanol solution for subsequent stirring operation at 60°C for 2 hours. The remainder of the extraction was removed by the above tree times extractions with 500 liters in total amount of ethanol solution. The extract solution free of the remainders was then condensed so that this condensed extract solution is, thereafter, subjected to a vacuum drying process to obtain about 1.2 kg of the extract from *Cassia mimosoides* L. var. *nomame* Makino.

### EXAMPLE 2 :

The same extraction operations as in Example 1 were carried out to obtain a condensed extract solution. 40 liters of this condensed extract solution was then transferred into a distillation kettle of 200 liters for a distillation for 2 hours with a steam at 2.0kg/cm² and 30 litcrs per a hour in order to remove volatile components from the steam-distillated extract. The remainder was then added with 400 liters of water of a temperature of not higher than 40°C for stirring the same and subsequent leaving the same prior to removal of a supernatant liquid before removal of the liquid component from the extract. A precipitate was dried by vacuum freeze-dry to obtain a dry powder. This dry powder was then transferred into a hot air oven to heat the powder at a temperature of 110°C for 3 hours, thereby obtaining 1.1 kg of the extract powder. It was confirmed that this extract powder is reduced in astringency and bittemess and suitable for addition into foods and drinks. The extract was subjected to a determination with Folin-Ciocaltcu reagent to confirm that a phenol contact was about 50% ((+)-catechin base). The extract was further subjected to a determination with hydrochloric acid-vanillin reagent to confirm a condensed tannin contact was about 14%.

### EXAMPLE 3 :

10 kg of mulberry leaf dry powder was immersed into 120 liters of 50%-ethanol solution for subsequent stirring operation at 50°C for 3 hours. This mixture solution was filtered so that a vacuum condensation to the filtrate was carried out for freeze-drying process, thereby to obtain 1.5kg of an extract from mulberry leaf. A measurement by gas chromatography was carried out to confirm that a content of 1-deoxynodirimycin in the extract was about 0.4%.

### EXAMPLE 4:

10 kg of mulberry leaf dry powder was immersed into 120 liters of 50%-cthanol solution for subsequent stirring operation at 50°C for 3 hours. This mixture solution was filtered so that a vacuum condensation to the filtrate was carried out for fractionation by chromatography and subsequent freeze-drying processes, thereby to obtain 175g of an cxtract from mulberry leaf. A measurement by gas chromatography was carried out to confirm that a contcnt of 1-deoxynodirimycin in the extract was about 7.4%.

### EXAMPLE 5 :

10 kg of mulberry leaf dry powder was immersed into 120 liters of 50%-ethanol solution for subsequent stirring opcration at 50°C for 3 hours. This mixture solution was filtered so that a vacuum condensation to the filtrate was carried out for addition of dextrin as excipient at 20% and subsequent freeze-drying processes, thereby to obtain 1.8kg of an cxtract from mulberry leaf. A measurement by gas chromatography was carried out to confirm that a content of 1-deoxynodirimycin in the extract was about 0.3%.

### EXAMINATION 1 :

Sprague-Dawley female rats with initial weights of 273-335g were used. The rats were individually reared in separate cages made of stainless steel at a temperature of 24±2°C in 12 hours day and 12 hours night cycle, wherein 12 hours day is 8:00-20:00.

The rats were divided into to groups, one is the comparative group and the other is dose group. In the comparative group, the rats were reared for twelve weeks with feeds shown on the following Table 1, wherein the rats are maintained to be free to intake the feeds and water.

**Table 1**

| Compositions of feeds | |
|---|---|
| Carbohydrate (corn starch, α-com starch, cyclose) | 56.9949 % |
| Protein (casein) | 18.0000 % |
| Fat (lard) | 20.0000 % |
| Mineral mixtures | 3.5000 % |
| Vitamin mixtures | 1.0000 % |
| L-cystine | 0.2511 % |
| Choline bitartrate | 0.2500 % |
| Ternary butyl hydroquinone | 0.0040 % |

The dose group was further divided into fourteen dose sub-groups. In the fourteen dose sub-groups group, the rats were reared for twelve weeks with intermediately fatty feeds added with compositions shown on the following Table 2, wherein the rats are maintained to be free to intake the feeds and water.

**Table 2**

| | *Cassia* extract (%) | mulberry leaf extract (%) |
|---|---|---|
| Comparative group | 0 | 0 |
| Dose sub-group 1 | 2 | 0 |
| Dose sub-group 2 | 4 | 0 |
| Dose sub-group 3 | 6 | 0 |
| Dose sub-group 4 | 0 | 1 |
| Dose sub-group 5 | 0 | 2 |
| Dose sub-group 6 | 0 | 3 |
| Dose sub-group 7 | 0 | 4 |
| Dose sub-group 8 | 0 | 5 |
| Dose sub-group 9 | 0 | 6 |
| Dose sub-group 10 | 2 | 1 |
| Dose sub-soup 11 | 2 | 2 |
| Dose sub-group 12 | 2 | 3 |
| Dose sub-group 13 | 2 | 4 |
| Dose sub-group 14 | 2 | 5 |

where *Cassia* extract is the extract extracted from *Cassia mimosoides* L. var. *nomame* Makino in Example 1, mulberry leaf extract is the extract extracted from mulberry leaf in Example 3.

After the rats had been reared, weights of the rats were measured to calculate an increment in weight of the rats from the initial weights. The rats were dissected to pick up fats under a skin of a frank and mesenteric fats to measure the weight of the picked up fats.

**Table 3**

| | *Cassia* extract (%) | mulberry extract (%) | weight increment (g) | skin fat (g) | mesenteric fat (g) |
|---|---|---|---|---|---|
| Comparative group | 0 | 0 | 111±14 | 10.2±1.2 | 14.9±1.3 |
| Dose sub-group 1 | 2 | 0 | 80±13 | 8.1±1.7 | 10.5±2.1 |
| Dose sub-group 2 | 4 | 0 | 76±11 | 7.9±1.9 | 10.1±2.5 |
| Dose sub-group 3 | 6 | 0 | 71±10 | 7.8±1.5 | 9.7±1.8 |
| Dose sub-group 4 | 0 | 1 | 98±7 | 9.2±0.4 | 13.9±1.6 |
| Dose sub-group 5 | 0 | 2 | 95±13 | 9.5±1.3 | 14.0±2.7 |
| Dose sub-stoup 6 | 0 | 3 | 94±12 | 9.6±1.0 | 13.6±1.4 |
| Dose sub-group 7 | 0 | 4 | 92±14 | 9.4±1.2 | 12.8±2.1 |
| Dose sub-group 8 | 0 | 5 | 91±9 | 9.2±0.9 | 12.5±1.6 |
| Dose sub-group 9 | 0 | 6 | 92±11 | 9.2±1.1 | 13.0±1.2 |
| Dose sub-group 10 | 2 | 1 | 79±11 | 7.6±0.8 | 9.8±1.0 |
| Dose sub-group 11 | 2 | 2 | 80±8 | 8.0±1.2 | 9.5±0.9 |
| Dose sub-group 12 | 2 | 3 | 80±9 | 8.5±1.5 | 9.2±1.3 |
| Dose sub-group 13 | 2 | 4 | 76±11 | 7.8±0.8 | 8.3=1.1 |
| Dose sub-group 14 | 2 | 5 | 78±10 | 7.9±0.9 | 8.6±1.0 |

where *Cassia* extract is the extract extracted from *Cassia mimosoides* L. var. *nomame* Makino in Example 1, mulberry extract is the extract extracted from mulberry leaf in Example 3, weight increment is the increment in weight of the rats from the initial weights, skin fat is the fats picked out under a skin of a frank and mcsenteric fat is the fats picked out from to mesenteric.

The rats in the dose sub-groups 1-3 supplied with the extract from *Cassia mimosoides* L. var. *nomame* Makino were apparently suppressed to increase the weight and were apparently reduced in skin fats viscus fats as compared to the rats in the comparative group. The rats in the dose sub-groups 4-9 supplied with the extract from mulberry leaf were slightly suppressed to increase the weight and wcre slightly reduced in skin fats viscus fats as compared to the rats in the comparative group. The rats in the dose sub-groups 10-14 supplied with both the extract from *Cassia mimosoides* L. var. *nomame* Makino and the extract from mulberry leaf were decreased in weight and skin fat and remarkably reduced in viscus fats as compared to the rats in the comparative group. It was confirmed that the dose of the extract from *Cassia mimosoides* L. var. *nomame* Makino in combination with the extract from mulberry leaf provides synergistic effects. In order to suppress the weight incrcase and reduce the viscus fats, the combined use of the above two extracts is effective. More effective mixture ratio in weight of the extract from *Cassia mimosoides* L. var. *nomame* Makino to the extract from mulberry leaf is in the range of 1 : 0.5 to 1 : 2.5. The optimum mixture ratio in weight of the extract from *Cassia mimosoides* L. var. *nomame* Makino to the extract from mulberry leaf is 1 : 2.

### EXAMPLE 6 :

In the same manner as in Example 3, an extract was extracted from mulberry leaf with a hot ethanol solution. 750g of an extract from *Cassia mimosoides* L var. *nomame* Makino prepared in Example 2 was dissolved into the above mulberry leaf extraction filtrate. This mixture solution was subjected to a vacuum condensation into about 30 liters for addition of 750g of dextrin and subsequent, thercby to obtain 3kg of a composition suitable to be added into foods and drinks as obesity-improver composition for reducing the viscus fats. This composition was in the form of fine powders with reduced astringency and bitterness which is suitable for addition into foods and drinks.

### EXAMPLE 7 :

2.0kg of cacao mass, 1.7kg of cacao butter, 2.0kg of whole milk powder, 4.2kg of sugar, and 50g of the composition obtained in Example 6 were put into a mixer for mixing at 38-43°C for 30 minutes before the mixture was then transferred into a refiner for refining operation at 35-36°C to obtain uniform particles of not larger than 40 micrometers in diameter. The particles were then transferred into a conching machinc for stirring and mixing operations at about 60°C for 40 hours as conching process. Immediately before the conching process has been finished, 50g of lecithin as an emulsifier and a small amount of essence were added for homogenization. A tempering was then carried out at 27-32°C for subsequent molding and cooling for solidification to obtain a chocolate. The chocolate has a good flavor and is suitable as an obesity-improver for reducing the viscus fats.

### EXAMPLE 8 :

500g of gum base, 1.2kg of sugar, 100g of corn syrup, 40g of citric acid, 20g of essence, and 30g of the composition obtained in Example 6 were admixed to form a chewing gum. This chewing gum has a good flavor and is suitable as an obesity-improver for reducing the viscus fats.

### EXAMPLE 9 :

300g of granulated sugar, 300g of shortening, 80g of margarine, 50g of corn starch, 70g of whole milk powder, 10g of salt, 10g of sodium bicarbonate, 1g of vanilla essence, and 50g of the composition obtained in Example 6 as well as 150g of water werc admixed by a mixer for aeration and subsequent addition of wheat flour to prepare a row material for biscuit. The row material was gill-spread and then molded before roasted in oven at 200°C for 10 minutes to form a soft biscuit. This soft biscuit has a good hardness and is suitable as an obesity-improver for reducing the viscus fats.

### EXAMPLE 10 :

220g of sugar, 168g of corn syrup, 72g of water were heated and dissolved for boiling at 145-150°C before admixing 12g of the composition obtained in Example 6 for subsequent molding to form a hard candy. This hard candy has a smooth-melting in mouth and is suitable as an obesity-improver for reducing the viscus fats.

### EXAMPLE 11 :

40g of lactose, 19g of corn starch, 1g of sugar ester, and 30g of the composition obtained in Example 6 were admixed and then extruded by an extruder-granulator for granulation before compression molding by punching machine to form 100 bare tablets of obesity-improving agent. A single tablet contains 300 mg of the extracts from *Cassia mimosoides* L. var. *nomame* Makino and from mulberry leaf.

### EXAMPLE 12 :

4.0kg of brown rice and 4.0kg of Coix werc mixed for subsequent extraction with hot water of 80-90°C for 5-10 minutes to extract a tea. The tea was then added with 99g of sodium carbonate, 153g of vitamin C and 4.5kg of the composition obtained in Example 6 to prepare 1000 liters of an obesity-improving tea. This tea has a good flavor and is suitable as an obesity-improver for reducing the viscus fats.

### EXAMPLE 13 :

2.6kg of 30%-fresh cream, 1.19kg of skim condcnsed milk, 524g of skim milk, 541g of sugar, 720g of corn syrup powder, 30g of emulsifier, 30g of stabilizer, 4.32kg of water, and 20g of the composition obtained in Example 6 were put into a mixer for mixing at 60-65°C before the mixture was then transferred into a homogenizer for homogenization at 60-65°C until fats become not larger than 2 micrometers to prepare an ice cream mix. This ice cream mix was then transferred into a bactericidal oven for placing the ice cream mix in thc bactericidal oven at 85-90°C for 15-30 seconds to sterilize the ice cream mix. The sterilized ice cream mix was then cooled at not higher than 5°C for an aging and subsequent transfer of the ice cream mix into the freezer to freeze the same at -2°C to -5°C with mixing with air to form a soft ice cream. The soft ice cream was further frozen at -20°C to form a hard ice cream. The soft and hard ice creams have a smooth-melting in mouth, a good flavor and is suitable as an obesity-improver for reducing the viscus fats.

### EXAMPLE 14 :

100g of sugar, 3g of cyclodextrin, 1.5g of sodium carbonate, 3g of cellulose, 3g of essence, 50g of coffee extract, and 20g of the composition obtained in Example 6 were dissolved into water so that a total volume becomes 1000 milliliters for dividing the same into 50 milliliters to prepare a liquid medicine. This liquid medicine has a good flavor is suitable as an obesity-improver for reducing the viscus fats.

Whereas modifications of the present invcntion will be apparent to a person having ordinary skill in the art, to which the invention pertains, it is to be understood that embodiments as shown and described by way of illustrations are by no means intendcd to be considered in a limiting sense. Accordingly, it is to be intended to cover by claims all modifications which fall within the spirit and scope of the present invention.

## Claims

1. A composition including a first extract from *Cassia mimosoides* L. var. *nomame* Makino and a second extract from mulberry.

2. The composition as claimed in claim 1, characterized in that a compositional ratio in weight of said first extract and said second extract is in the range of 1 : 0.5 to 1 : 2.5.

3. The composition as claimed in claim 1, characterized in that said first extract is roasted.

4. The composition as claimed in claim 3, characterized in that said first extract is roasted at 100-130°C for 1-5 hours.

5. Foods and drinks containing a first extract from *Cassia mimosoides* L. var. *nomame* Makino and a second extract from mulberry.

6. The foods and drinks as claimed in claim 5, characterized in that said first and second extracts are admixed into a base material for said foods and drinks.

7. The foods and drinks as claimed in claim 5, characterized in that a ratio in weight of said first extract and said second extract is in the range of 1 : 0.5 to 1 : 2.5.

8. The foods and drinks as claimed in claim 5, characterized in that said first extract is roasted.

9. The foods and drinks as claimed in claim 8, characterized in that said first extract is roasted at 100-130°C for 1-5 hours.

10. An obesity-improving agent including a composition comprising a first extract from *Cassia mimosoides* L. var. *nomame* Makino and a second extract from mulberry.

11. The obesity-improving agent as claimed in claim 10, characterized in that a compositional ratio in weight of said first extract and said second extract is in the range of 1 : 0.5 to 1: 2.5.

12. The obesity-improving agent as claimed in claim 10, charactcrized in that said first extract is roasted.

13. The obesity-improving agcnt as claimed in claim 12, characterized in that said first extract is roasted at 100-130°C for 1-5 hours.

14. A method of preparing a composition comprising the steps of:
preparing a first extract from *Cassia mimosoides* L. var. *nomame* Makino and a second extract from mulberry ; and
admixing said first and second extracts.

15. The method as claimed in claim 14, characterized in that said first and said second extracts are admixed at a ratio in weight in the range of 1 : 0.5 to 1 : 2.5.

16. The method as claimed in claim 14, further comprising the step of roasting said first extract before admixed with said second extract.

17. The method as claimed in claim 16, characterized in that said first extract is roasted at 100-130°C for 1-5 hours.

18. A method of preparing foods and drinks comprising the steps of:
preparing a first extract from *Cassia mimosoides* L. var. *nomame* Makino and a second extract from mulberry;
admixing said first and second extracts to prepare a composition ; and
adding said composition into foods and drinks.

19. The method as claimed in claim 18, characterized in that said composition is admixed into a base material for said foods and drinks.

20. The method as claimed in claim 18, characterized in that said first and said second extracts are admixed at a ratio in weight in the range of 1 :0.5 to 1 :2.5.

21. The method as claimed in claim 18, further comprising the step of roasting said first extract before admixed with said second extract.

22. The foods and drinks as claimed in claim 21, characterized in that said first extract is roasted at 100-130°C for 1-5 hours.

23. A method of preparing an obesity-improving agent comprising the steps of:
preparing a first extract from *Cassia mimosoides* L. var. *nomame* Makino and a second extract from mulberry ;
admixing said first and second extracts ;
forming said admixture into an obesity-improving agent.

24. The method as claimed in claim 23, characterized in that said first and said second extracts are admixed at a ratio in weight in the range of 1 : 0.5 to 1 : 2.5.

25. The method as claimcd in claim 23, further comprising the step of roasting said first extract before admixed with said second extract.

26. The method as claimed in claim 25, characterized in that said first extract is roasted at 100-130°C for 1-5 hours.
